Europäisches Patentamt

European Patent Office

Office européen des brevets

(11) Numéro de publication : **0 357 488 B1**

(12) **FASCICULE DE BREVET EUROPEEN**

(45) Date de publication du fascicule du brevet :
09.12.92 Bulletin 92/50

(51) Int. Cl.⁵ : **C07C 2/84,** C01B 3/02,
C01C 1/04, C07C 273/10

(21) Numéro de dépôt : **89402316.7**

(22) Date de dépôt : **21.08.89**

(54) *Procédé de production couplée d'oléfines et d'ammoniac à partir du gaz naturel et utilisation de ce procédé pour la production d'urée.*

(30) Priorité : **25.08.88 FR 8811312**

(43) Date de publication de la demande :
**07.03.90 Bulletin 90/10**

(45) Mention de la délivrance du brevet :
**09.12.92 Bulletin 92/50**

(84) Etats contractants désignés :
**AT BE CH DE ES GB IT LI LU NL**

(56) Documents cités :
**EP-A- 0 336 823**

(73) Titulaire : **INSTITUT FRANCAIS DU PETROLE
4, Avenue de Bois Préau
F-92502 Rueil-Malmaison (FR)**

(72) Inventeur : **Cameron, Charles
28, rue Henri Barbuse
F-75005 Paris (FR)**
Inventeur : **Dang Vu, Quang
48 bis, Boulevard du Général Leclerc
F-92200 Neuilly (FR)**
Inventeur : **Le Page, Jean-François
13, rue des Primevères
F-92500 Rueil-Malmaison (FR)**
Inventeur : **Mimoun, Hubert
34, rue Hippolyte Bisson
F-92500 Rueil-Malmaison (FR)**

**Description**

La présente invention décrit un procédé de production couplée d'oléfines et d'ammoniac à partir du gaz naturel. Elle décrit également l'utilisation de l'ammoniac obtenu pour la fabrication d'urée.

Le couplage oxydant du méthane est une réaction aujourd'hui largement décrite et peut être réalisé en mode séquentiel ou en mode simultané pour produire de l'éthylène et d'autres hydrocarbures.

La réaction du couplage oxydant en mode séquentiel consiste en l'oxydation du méthane par un agent réductible suivie de la réoxydation de cet agent, séparément, par l'oxygène de l'air. On a mentionné dans plusieurs brevets américains (voir, par exemple, US-4 499 323, 4 523 049, 4 547 611, 4 567 307) l'utilisation de nombreux oxydes de métaux, notamment les oxydes de Mn, Ce, Pr, Sn, In, Ge, Pb, Sb, Bi, Tb, comme agents réductibles pour cette réaction.

La réaction du couplage oxydant en mode simultané (balayage d'un mélange de méthane et d'oxygène sur une masse de contact) peut s'écrire qualitativement :

$$CH_4 + O_2 \rightarrow C_2H_6 + C_2H_4 + \text{autres hydrocarbures} + CO + CO_2 + H_2 + H_2O$$

Il a été mentionné dans plusieurs brevets (voir, par exemple, brevets européens EP 210 383, EP 189 079, EP 206 044 et brevet mondial W0 86 07351) l'utilisation d'oxydes de métaux des terres rares, d'oxydes de métaux alcalins et alcalino-terreux et d'oxydes de titane, zirconium, hafnium et zinc soit seuls, soit mélangés, comme catalyseurs pour la réaction du couplage oxydant du méthane en mode simultané.

Sur le plan du principe, chaque mode de couplage oxydant a ses avantages et ses inconvénients. Un des avantages du mode séquentiel est de pouvoir utiliser directement l'air pour la séquence d'oxydation du catalyseur réduit dans la mesure ou l'on réalise après la séquence de couplage une purge intermediaire par de la vapeur d'eau. L'inconvénient majeur réside dans le caractère séquentiel de l'opération.

L'avantage majeur du mode simultané réside donc dans la continuité de l'opération, spécialement si elle se déroule dans un réacteur à lit fluidisé ou à lit circulant permettant une injection continue de catalyseur frais pour compenser les pertes mécaniques de catalyseur et toute décroissance éventuelle de son activité chimique. Un inconvénient majeur est lié à la nécesité d'utiliser de l'oxygène comme agent oxydant de préférence à l'air. En effet, si on se réfère au nombre d'atomes de carbone de méthane ayant réagi, on ne peut, avec les catalyseurs utilisés dans ce couplage oxydant, obtenir une sélectivité supérieure à 80% pour une conversion maximum de 25%. Le fait d'utiliser de l'air comme agent oxydant entraînerait donc la nécessité de séparer l'azote en fin d'opération alors qu'il se trouve mélangé avec du méthane et de l'hydrogène qu'il importe, pour des raisons économiques, de recycler ou de récupérer : il convient donc dans ce cas de réaliser au préalable une séparation de l'oxygène et de l'azote afin d'opérer la réaction de couplage oxydant sinon en présence d'oxygène pur du moins en présence d'un oxygène très appauvri en azote.

Cet inconvénient de devoir réaliser au préalable une séparation de l'oxygène et de l'azote peut cependant se présenter comme un avantage dans la mesure où l'on conduit l'opération de manière à pouvoir tirer parti de l'azote disponible. De plus, si on n'a pas besoin d'un courant d'hydrogène très appauvri en azote, on n'a plus besoin d'une séparation préalable de l'oxygène et de l'azote.

La présente invention propose donc une transformation du gaz naturel impliquant de combiner :

- un couplage oxydant du méthane selon le mode simultané,
- une déshydrogénation des hydrocarbures aliphatiques comprenant au moins 2 d'atomes de carbone. La mise en oeuvre de cette réaction, très endothermique, permet de tirer le meilleur avantage des calories dégagées durant le couplage oxydant globalement exothermique,
- une synthèse de l'ammoniac et/ou de l'urée qui permet d'utiliser au mieux les sous produits de l'opération, à savoir le gaz carbonique, l'hydrogène et l'azote.

La présente invention concerne plus particulièrement un procédé de production d'oléfines et d'ammoniac à partir de gaz naturel renfermant du méthane et de l'éthane, caractérisé en ce que :

**a)** on sépare le gaz naturel en au moins deux fractions, une première fraction de gaz enrichie en méthane et une deuxième fraction de gaz enrichie en hydrocarbures supérieurs au méthane,

**b)** on oxyde la première fraction de gaz provenant du gaz naturel par un gaz renfermant de l'oxygène en proportion de 0,05 à 0,4 mole par mole de méthane, en présence d'une masse de contact permettant le couplage oxydant du méthane en hydrocarbures supérieurs,

**c)** on mélange l'effluent de l'étape (b) avec la fraction de gaz enrichie en hydrocarbures supérieurs de l'étape (a) quand au moins 80% de l'oxygène moléculaire a déjà été consommé dans l'étape (b),

**d)** on pyrolyse le mélange résultant de l'étape (c), de manière à déshydrogéner lesdits hydrocarbures supérieurs et à obtenir un mélange renfermant des oléfines, de l'hydrogène et éventuellement de l'azote,

**e)** on fractionne l'effluent de l'étape (d) afin d'obtenir une fraction riche en hydrogène,

**f)** on ajuste les proportions d'hydrogène et d'azote de la fraction riche en hydrogène, de manière à permettre la synthèse d'ammoniac, et

**g)** on fait réagir le mélange de l'étape (f), en présence d'un catalyseur pour la synthèse d'ammoniac, afin d'obtenir un courant de gaz renfermant de l'ammoniac.

Lorsqu' on veut utiliser cet ammoniac pour produire de l'urée, on fait réagir l'ammoniac obtenu dans l'étape (g) avec au moins un partie du dioxyde de carbone obtenu dans l'étape (b) afin d'obtenir de l'urée.

Par rapport au procédé connu de couplage oxydant du méthane, le procédé permet d'augmenter les rendements en oléfines et en hydrogène en utilisant une réaction de pyrolyse sur le mélange de l'effluent de l'étape d'oxydation et de la fraction de gaz enrichie en éthane et autres hydrocarbures supérieurs. En outre, ce procédé permet de transformer l'hydrogène résultant de la réaction d'oxypyrolyse du gaz naturel et une partie de l'azote, provenant du gaz naturel et/ou de l'air, en ammoniac.

Il est préférable de régler les opérations d'oxypyrolyse du gaz naturel de façon à donner un rendement maximum en oléfines tout en produisant un rapport molaire $H_2/CO_2$ compris entre environ 3 et 9; c'est à dire au voisinage de la stoechiomètrie régissant la synthèse de l'urée. Il est alors possible, dans le procédé, de réaliser avec l'ammoniac et le dioxyde de carbone la synthèse de l'urée.

Les exemples suivants, non limitatifs, illustrent les différents modes de réalisation qui peuvent être employés pour atteindre la production couplée d'oléfines et d'ammoniac à partir du gaz naturel.

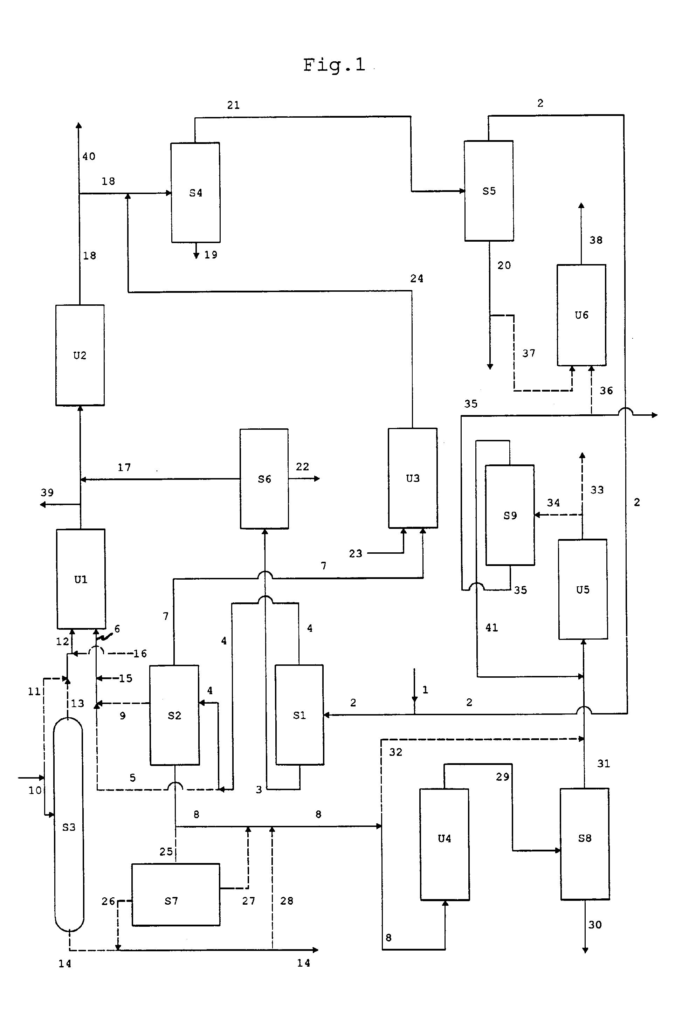

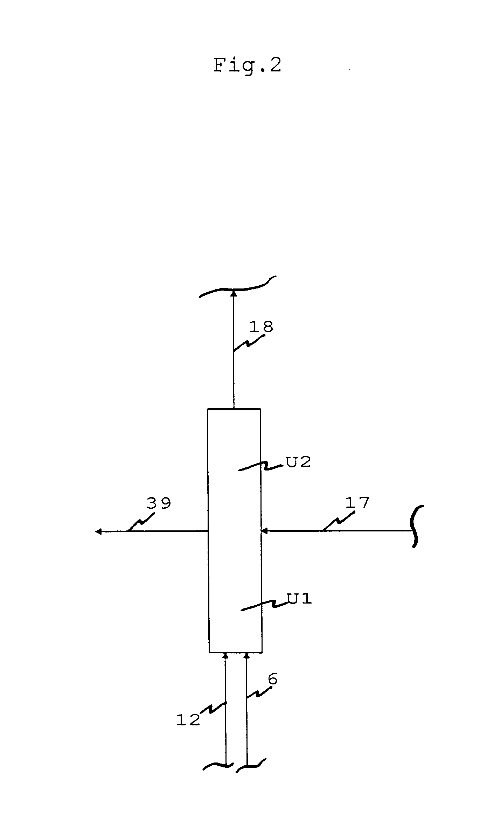

Les mises en oeuvre préférées de la présente invention sont représentées schématiquement dans les figures 1 et 2 et sont décrites ci-dessous. Il faut noter que les unités de chauffage, refroidissement, compression et détente ont été omises pour simplifier les figures, mais leurs nécessités et positions sont tout à fait évidentes pour l'homme de l'art.

La figure 1 illustre un premier mode de mise en oeuvre.

La figure 2 illustre une modification du mode de mise en oeuvre de la figure 1.

Le gaz naturel est tout d'abord refroidi avant de subir les traitements usuels de déshydratation, désulfuration et décarbonatation. Le courant de gaz naturel purifié (1), qui entre dans la ligne (2), est conduit au séparateur (S1) puis fractionné, par exemple par condensation ou par extraction par solvant sélective, en hydrocarbures $C_2^+$ (3) et en gaz substantiellement exempt d'hydrocarbures supérieurs (4). Le courant de gaz (4), qui est constitué principalement de méthane, peut être utilisé tel quel (5) ou soumis à une autre étape de séparation (S2) pour enlever le monoxyde de carbone (7), l'azote et l'hydrogène (8) du méthane (9). Le courant d'air (10) peut être aussi utilisé tel quel (12) ou fractionné (S3) en oxygène appauvri d'azote (13) et en azote (14). Quelle que soit la forme particulière du procédé choisi, les courants de méthane (6) et d'oxygène (12) sont préchauffés, soit séparément soit ensemble et avec ou sans vapeur d'eau qui est additionnée via les lignes (15) et (16), avant d'être introduits dans le réacteur d'oxydation (U1). L'utilisation de la vapeur d'eau permet de préchauffer le méthane avec moins de risque de décomposition en carbone et hydrogène. Quand la vapeur d'eau est utilisée, le rapport en poids vapeur d'eau/méthane est de préférence compris entre environ 0,2 et 1.

La température initiale du mélange réactif dans le réacteur dépend largement du type de réacteur utilisé, mais est généralement située entre 400 et 750°C. La réaction est effectuée à une température d'environ 800 à 1400°C. La pression dans le réacteur est comprise entre 1 et 50 bars, de préférence entre environ 1 et 10 bars.

Diverses formules de masse de contact peuvent être mises en oeuvre pour initier la réaction du couplage oxydant dans la zone (U1) du procédé, par exemple :

- des masses de contact contenant au moins un mélange d'oxydes et/ou de carbonates de deux ou plusieurs métaux alcalino-terreux,
- des masses de contact contenant au moins des oxydes et/ou des sulfates et/ou des phosphates et/ou des carbonates de titane, zirconium, hafnium, manganèse, cérium, étain, indium, germanium, plomb, antimoine, zinc, bismuth, chrome et nickel préférentiellement avec un ou plusieurs oxydes et/ou carbonates de métaux alcalins, alcalino-terreux, terres rares et silice,
- des masses de contact contenant au moins des oxydes et/ou des carbonates et/ou des oxycarbonates de métaux de terre rare et de métaux alcalino-terreux,
- des masses de contact contenant au moins des phases de cérium tels que $SrCeO_3$, $BaCeO_3$, $Sr_2CeO_4$ et $Ba_2CeO_4$.

Il faut noter que les masses de contact, qu'on peut utiliser pour initier la réaction du couplage oxydant, ne sont toutefois pas limitées aux exemples montrés ci-dessus. Bien entendu, on peut utiliser toute masse de contact connue pour cet usage.

La masse de contact peut être par exemple sous forme de billes, de monolithes imprégnés, de fibres imprégnées ou de concassés, en fonction du type de réacteur utilisé, qui peut être par exemple un réacteur à lit fixe, à lit bouillonnant ou à lit circulant.

La réaction d'oxydation partielle, réalisée dans la section (U1), est fortement exothermique et, pour éviter une élévation de température trop importante qui nuirait à la sélectivité de l'opération, on opère avec des rapports molaires oxygène/méthane inférieurs à 0,4 et de préférence inférieurs à 0,2.

La réaction de couplage oxydant peut s'opérer en lit fixe, en lit bouillonnant, mais la mise en oeuvre préférée du catalyseur, dans la présente invention, consiste à opérer en lit circulant avec un catalyseur de granulométrie comprise entre 10 et 200 $\mu$m et de préférence entre 40 et 120 $\mu$m. Le temps de résidence du méthane, pour cette mise en oeuvre dans la zone de réaction (U1) reste compris entre 0,01 et 50 secondes et de préférence entre 0,05 et 5 secondes. Le rapport des débits horaires pondéraux de catalyseur et de méthane est maintenu, de manière à ce qu'une partie notable de la chaleur de réaction puisse être captée sous forme de chaleur sensible emmagasinée par le catalyseur solide; à la sortie de la zone réactionnelle cette chaleur sensible peut être utilisée pour la production de vapeur à haute pression, de manière à ramener la température de la masse de contact au voisinage de 500-750°C. Ce débit rapide de masse de contact dans la zone réactionnelle (U1) permet de modérer l'élévation de température au fur et à mesure que se consomme l'oxygène dans la zone d'oxydation.

Pour aider à contrôler, en fin d'oxydation, l'élévation de température et surtout pour tirer profit de l'exothermicité du couplage oxydant, il est avantageux d'introduire dans le réacteur d'oxypyrolyse, en un endroit où au moins environ 80% (de préférence au moins 95%) de l'oxygène moléculaire ont déjà été consommés lors de l'oxydation sélective du méthane, la fraction d'hydrocarbures supérieurs (17) provenant du gaz naturel et éventuellement provenant d'une étape précédente d'oxydation sélective du méthane. Ladite fraction (17) peut être introduite à température ambiante ou préchauffée à une température inférieure à la température de l'effluent. Dans les deux cas, la fraction (17) sert à abaisser la température de l'effluent, et produit alors un mélange qui est à une température inférieure à la température de l'effluent.

L'addition de la fraction (17) après l'étape de l'oxydation sélective du méthane permet la transformation de la fraction d'hydrocarbures supérieurs (17) en oléfines et hydrogène, ce qui n'est pas le cas si les hydrocarbures supérieurs traversent l'unité d'oxydation (U1). Comme les vitesses relatives d'oxydation des alcane $C_2{}^+$, dans les conditions de couplage oxydant du méthane, sont environ 15 à 100 fois plus grandes que celles de l'oxydation du méthane, les alcanes $C_2{}^+$ sont transformés en oléfines et eau bien avant que le méthane ne commence à réagir. Ainsi, l'addition de la fraction (17) après l'étape de l'oxydation sélective du méthane permet la récupération d'hydrogène au lieu d'eau.

La fraction (17) et l'effluent de la réaction d'oxydation, qui contient de l'éthane comme produit sélectif principal, sont mélangés et la déshydrogénation endothermique de ces hydrocarbures est effectuée en traversant la section de pyrolyse (U2). Le temps de résidence du mélange (de l'effluent de l'unité (U1) et de la fraction (17)) est fixé en fonction de la température du mélange et de la sévérité de la pyrolyse désirée, mais est généralement compris entre environ 0,001 et 1 seconde et la température entre environ 800 et 1400°C. Le temps de résidence et la température, dans la zone (U2), sont réglés en fonction du rapport alcanes/oléfines désiré. La réaction de pyrolyse peut être effectuée soit en absence de masse de contact soit en présence d'une masse de contact telle que l'une de celles antérieurement décrites pour l'oxydation sélective du méthane.

Dans le cas d'un lit circulant, mettant en oeuvre un courant ascendant de catalyseur pulvérulent, sous forme de microbilles par exemple, cette zone (U2) correspond à la partie supérieure de la conduite de remontée du catalyseur ("riser") et à la zone de désengagement de ce même catalyseur impliquant l'ensemble du ballon de collecte du catalyseur depuis la sortie de la zone de strippage jusqu'à la sortie des cyclones (zone de phase diluée), la partie inférieure du "riser" étant utilisée comme zone (U1). Le catalyseur strippé est refroidi par échange jusqu'à une température comprise entre par exemple 600 et 750°C avant d'être recyclé au réacteur. La zone d'échange peut être constituée des tubes d'une chaudière utilisée pour produire la vapeur nécessaire au procédé ou des tubes d'un échangeur servant à préchauffer les charges hydrocarbonées.

Le gaz qui sort de la section (U2), et, qui contient $H_2O$, $CO_2$, $CO$, $CH_4$, $(N_2)$, $H_2$ ainsi qu'une fraction $C_2{}^+$ composée pour l'essentiel d'éthylène, d'éthane et de propylène, est alors envoyé par la ligne (18) dans un train de séparation où l'on sépare successivement l'eau (19), le gaz carbonique (20) et les divers hydrocarbures de la fraction $C_2{}^+$ (3) par diverses méthodes de séparation, telles que :
- Lavage aux amines, permettant d'éliminer $CO_2$
- absorption par liquides hygroscopiques ou par solides réactifs permettant d'éliminer $H_2O$ et/ou $CO_2$
- adsorption sur des solides permettant d'éliminer $H_2O$ et/ou $CO_2$ et/ou $CO$
- fractionnement par condensation sous pression à basse température permettant de séparer $H_2O$, $CO_2$ et les hydrocarbures $C_2{}^+$, ou
- n'importe quelles méthodes permettant de séparer l'eau, le gaz carbonique et les divers hydrocarbures de la fraction $C_2$,

comme cela est décrit dans l'ouvrage "Gas Purification" Fourth Edition, A.L. Kohl et F.C. Riesenfeld, (1985) Gulf Publishing Company, Houston.

L'effluent (18) traverse le séparateur (S4), est envoyé par la ligne (21) au séparateur (S5), puis transféré via la ligne (2) au séparateur (S1) d'où il sort appauvri en $H_2O$, $CO_2$, et en hydrocarbures supérieurs. L'effluent (4), qui contient principalement du $CH_4$, $H_2$, $CO$ et éventuellement de l'azote, peut être fractionné en trois parties:

4

1) un courant riche en méthane (9), 2) un courant riche en hydrogène et en azote (8) et 3) un courant riche en CO (7) par une combinaison de diverses méthodes de séparations, telles que :

- absorption par des complexes de cuivre, permettant de séparer CO,
- fractionnement par condensation sous pression à basse température, permettant de séparer le méthane d'une part et l'hydrogène et l'azote d'autre part,
- séparation par membranes perméables gazeuses, permettant de séparer le méthane et l'azote d'une part et l'hydrogène d'autre part,
- séparation par "pressure swing absorption", permettant de séparer l'hydrogène des mélanges gazeux contenant de l'hydrogène, du méthane et/ou de l'azote et/ou de l'oxyde de carbone,
- séparation par des métaux capables de capturer l'hydrogène tels que l'uranium, le magnésium, le titane ou le lanthane, ou les alliages Ni-Mg ou Ni-La, permettant de séparer l'hydrogène par le chauffage des hydrures formés, ou
- toute combinaison de diverses méthodes connues pour la séparation des gaz présents dans la ligne (4), l'effluent qui sort du séparateur (S1).

Ces fractionnements sont globalement représentés par l'unité (S2) et les gaz séparés sont éliminés par les lignes (7), (8) et (9).

Le gaz renfermant des hydrocarbures $C_2$ est envoyé au séparateur (S6) ou il est fractionné par n'importe quelle méthode qui permet la séparation des hydrocarbures saturés (17); ces derniers sont injectés dans le réacteur d'oxypyrolyse, en un endroit où ou moins environ 80% de l'oxygène moléculaire ont déjà été consommés. L'unité (S6) peut être toute unité capable de séparer des oléfines. Ce peut être une absorption dans l'acide sulfurique, une distillation, une oligomérisation des oléfines en carburants liquides, par exemple en essence et/ou en gas oil, une réaction de dimérisation en butène-1. Ces produits sont déchargés par la ligne (22).

Le courant riche en CO (7) peut être envoyé à l'unité (U3) où il est converti en $CO_2$ et $H_2$ par réaction avec la vapeur d'eau. La vapeur d'eau est envoyée à l'unité (U3) via la ligne (23). Cette réaction est effectuée, en général, selon les méthodes suivantes : à haute température, entre environ 330 et 500°C, et en présence de catalyseur oxyde de fer, oxyde de chrome ou oxyde de cobalt, oxyde de molybdène; à basse température, entre environ 180 et 280°C, et en présence de catalyseur oxyde de cuivre, oxyde de zinc-oxyde d'aluminium. les produits, $CO_2$ et $H_2$, de même que le CO et le $H_2O$ non convertis, sont injectés dans le train de séparation par la ligne (24).

Le courant de gaz qui contient l'hydrogène et l'azote (8) est traité en fonction du rapport molaire $H_2/N_2$. Pour des courants de gaz (8) avec un rapport molaire $H_2/N_2$ plus faible que 3, au moins une partie du courant est transférée vers la ligne (25) à un séparateur à membrane perméable gazeuse (S7) où la plus grande partie de l'azote est enlevée par la ligne (26). Le courant riche en hydrogène (27) rejoint la ligne (8) pour obtenir un rapport molaire $H_2/N_2$ d'au moins 3. Dans le cas où le courant (8) sortant du séparateur (S2) a déjà un rapport molaire $H_2/N_2$ d'au moins 3, l'unité de séparation (S7) n'est pas nécessaire.

Le courant de gaz avec un rapport molaire $H_2/N_2$ d'au moins 3 (8) est ensuite mélangé avec l'azote de la ligne (28) pour obtenir un rapport molaire $H_2/N_2$ de 3. Ce mélange est purifié par une réaction de méthanation, dans l'unité (U4), pour enlever les traces de CO puis transféré vers la ligne (29) et déshydraté dans l'unité de séparation (S8) pour enlever les traces d'eau qui sont sont déchargées par la ligne (30). Le mélange d'un rapport molaire $H_2/N_2$ égal à 3 est envoyé au réacteur de synthèse d'ammoniac (U5) par la ligne (31).

Dans le cas où le courant de gaz avec un rapport molaire $H_2/N_2=3$ ne contient pas de traces de CO ou d'eau, le courant de gaz est tranféré dans la ligne (32) puis (31) avant d'arriver à l'unité (U5).Un rapport $H_2/N_2$ légèrement différent de 3 peut être utilisé.

La synthèse d'ammoniac est une réaction bien connue qui se déroule à une pression entre environ 20 et 800 bars, de préférence entre 20 et 300 bars, et une température entre environ 200 et 600°C. Le catalyseur utilisé pour cette réaction peut être n'importe lequel connu pour cet usage, par exemple les catalyseurs décrits dans l'ouvrage "Ullman's Encyclopedia of Industrial Chemistry" Volume 2A, pp 143-242. D'un intérêt particulier sont les catalyseurs composés de fer métallique-alpha avec des oxydes non réductibles tels que les oxydes de potassium, calcium, aluminium et silicium.

L'effluent de l'unité (U5), renfermant de l'ammoniac, est déchargé par la ligne (33), ou peut être avantageusement transféré au séparateur (S9) via la ligne (34), où il est fractionné en ammoniac pur (35) et azote/hydrogène (41). Le courant de azote/hydrogène (41) est envoyé à la ligne (31) pour être éventuellement remis en contact avec le catalyseur dans l'unité (U5). L'ammoniac (35) peut être utilisé tel quel ou envoyé dans la conduite (36) à l'unité de synthèse d'urée (U6).

On peut aussi, dans une modification de l'invention, utiliser le $CO_2$ comme sous-produit dans l'étape d'oxydation (b). Comme des quantités importantes de $CO_2$ sont formées, il est avantageux de transformer au moins une partie du $CO_2$ en un produit à valeur ajoutée, et rendre alors l'utilisation du gaz naturel globalement plus avantageuse. Pour atteindre cet objectif, au moins une partie du $CO_2$ dans la conduite (37) et au moins une fraction de l'ammoniac dans la conduite (36) sont envoyés à l'unité (U6) pour la synthèse thermique d'urée.

L'unité (U6) opère généralement entre environ 100 et 300 bars, de préférence entre environ 150 et 250 bars, à une température comprise entre environ 180 et 300°C en utilisant un rapport molaire $NH_3/CO_2$ entre environ 2 et 6. On recueille le mélange contenant l'urée par la ligne (38).

## EXEMPLES

Un courant de gaz naturel (1) est fractionné dans le séparateur (S1) en méthane (4) et hydrocarbures supérieurs (3). Un courant d'air (10) est également fractionné dans le séparateur (S3) en oxygène (13) et azote (14). Le courant de méthane, qui est envoyé par la ligne (5) puis la ligne (6), alimente un tube vertical. Le réacteur reçoit aussi l'oxygène par la ligne (12). Dans la partie inférieure du tube (U1) on dispose le lit de contact.

On ajoute alors le courant (17) d'éthane et hydrocarbures supérieurs et le mélange résultant poursuit son cheminement dans la partie supérieure du tube (U2).

Les deux sections successives sont disposées chacune dans un four et la partie de canalisation entre les deux fours est isolée thermiquement.

Le gaz effluent (18) entre dans un train de séparation qui est globalement représenté par les séparateurs (S4), (S5), (S1), (S2) et (S7). L'hydrogène purifié provenant du séparateur (S7) est comprimé et l'azote, qui est envoyé par la ligne (28), est mélangé à l'hydrogène pour obtenir un mélange $H_2/N_2$ égal à 3. Le mélange $H_2/N_2 = 3$ alimente un réacteur en acier inoxydable via la ligne (32) puis la ligne (31).

Le gaz effluent sortant de l'unité (U5) est déchargé du réacteur par la ligne (33) et envoyé au séparateur (S9) via la conduite (34). L'ammoniac (35) est envoyé au réacteur (U6) via la ligne (36) et le $CO_2$ est envoyé au réacteur (U6) via la ligne (37). L'effluent du réacteur (U6) est déchargé par la ligne (38).

Les lignes (39) et (40) permettent de prélever des échantillons, notamment pour mesurer la conversion de l'oxygène et la composition du gaz provenant de l'unité (U2).

Dans la suite on appellera premier réacteur et second réacteur respectivement les sections inférieure oxydante (U1) et supérieure pyrolytique (U2) du tube ci-dessus.

## EXEMPLES 1-4

On opère avec un gaz naturel ayant un rapport volumique $C_2H_6/(C_2H_6+CH_4)=9,1\%$. Le lit de contact contient 0,3 g d'un mélange mécanique de $SrCO_3$ et $La_2(CO_3)_3$ dans un rapport atomique Sr/La de 0,5, qui a été calciné à 600°C, dilué dans 3,2 ml de grains d'alumine tabulaire.

Le temps de résidence dans le lit de contact de la section inférieure oxydante (U1) (dans les exemples 1, 2 et 4) est de l'ordre de 0,042 sec. Cette valeur est basée sur le volume (3,2 ml) et la température (880°C) du lit de contact et le débit de gaz à la sortie de ce lit (2,89 mol/h).

Dans l'exemple 3, ce gaz est envoyé en totalité, sans fractionnement, à travers les deux réacteurs (U1 puis U2).

Dans les autres cas (exemple 1, 2 et 4), le gaz est d'abord fractionné en méthane et éthane. Dans l'exemple 1, le méthane est envoyé au réacteur (U1), le réacteur (U2) n'étant pas utilisé. Dans l'exemple 2, le méthane traverse successivement les deux réacteurs (U1 puis U2); l'éthane n'est utilisé ni dans l'exemple 1 ni dans l'exemple 2.

Dans l'exemple 4, selon l'invention, le méthane traverse successivement les réacteurs (U1 et U2) et l'éthane est introduit à l'entrée du second réacteur (U2), en un point où la conversion de l'oxygène est de 99,7%. Dans tous les cas, on utilise un rapport $O_2/CH_4$ de 15 % en mole à l'entrée du réacteur (U1).

Le temps de résidence dans la section supérieure pyrolytique (U2) de cet exemple est de l'ordre de 0,74 sec. Le temps de résidence est calculé en utilisant le volume de la section (U2) dans le four et le débit par seconde du gaz qui sort du réacteur (U2) à une température de 880°C.

Les conditions opératoires et les résultats (ligne 40) sont donnés dans le tableau 1.

On montre que les débits d'hydrogène et de $C_2^+$ de l'exemple 4 sont considérablement plus forts que ceux obtenus dans l'exemple 3. Ceci montre l'effet positif de l'injection de l'éthane après l'endroit où la plupart de l'oxygène a été consommée. On montre aussi que le rapport molaire $H_2/CO_2$ de l'exemple 4 est 3,1; c'est à dire au voisinage de la stoechiométrie régissant la synthèse de l'urée. Le rapport $H_2/CO_2$ de l'exemple 3 est 2,2.

## EXEMPLE 5

L'exemple 5 est effectué dans les mêmes conditions que les exemples 1-4; toutefois la température du second réacteur est de 880°C et le lit de contact consiste de 3,2 ml de grains de magnésie imprégnés de

Ba(NO$_3$)$_2$ et de La(NO$_3$)$_3$ dans un rapport atomique de 0,5. Les débits (mol/h) des produits principaux après le séparateur (S4), un condenseur maintenu à 0°C, sont les suivants : H$_2$ (0,387), O$_2$ (0,001), CO (0,020), CO$_2$ (0,113), C$_2$H$_4$ (0,320), C$_2$H$_6$ (0,050), C$_3$H$_6$ (0,014).

## EXEMPLE 6

Le gaz effluent de l'exemple 5, sortant du séparateur (S4), est fractionné pour obtenir de l'hydrogène pur. Le gaz effluent (21) est envoyé successivement au séparateur (S5) (un condenseur maintenu à -78°C), au séparateur (S1) (un condenseur maintenu à -160°C), puis au séparateur (S2).

Le séparateur (S2) utilise une combinaison de deux méthodes de séparation, dont la première consiste à faire passer le gaz dans un condenseur maintenu à -196°C et la seconde consiste à faire passer le gaz contenant de l'hydrogène, sortant de la première étape, sur une masse de captation d'hydrogène. La masse de captation utilisée est de la poudre d'uranium.

La poudre d'uranium est chargée dans un réacteur en acier inoxydable. Le réacteur est mis sous vide, puis le gaz contenant de l'hydrogène est mis en contact avec la poudre. La température à l'intérieur du réacteur est maintenue entre environ 20 et 50°C pour que la poudre d'uranium soit transformée en hydrure d'uranium, UH$_3$. Quand la pression à l'intérieur du réacteur atteint à peu près 50 kPa, le courant du gaz contenant de l'hydrogène est arrêté et le réacteur est remis sous vide. Un courant d'hydrogène pur (8) est récupéré en chauffant le réacteur à 450°C. Cette procédure est répetée autant de fois qu'il est nécessaire pour obtenir la quantité suffisante d'hydrogène pur pour l'étape suivante.

Le courant d'hydrogène pur (8) est mélangé avec le courant d'azote pur (28) pour obtenir un rapport molaire H$_2$/N$_2$ égal à 3, envoyé dans la conduite (32), puis transféré dans la ligne (31).

Le courant (31), comprimé à 150 bars, alimente un réacteur (U$_5$) en acier inoxydable contenant 20 ml d'un catalyseur (composé de 93% (en poids) oxydes de fer, 3% Al$_2$O$_3$, 0,5% MgO, 0,5% SiO$_2$, 0,5% K$_2$O et 2,5% CaO) qui a été préréduit avec de l'hydrogène. Le débit du gaz réactif est de 1500 ml/min et la température est maintenue à 480°C. Le courant du gaz (33) sortant du réacteur (U5) contient 16,4% d'ammoniac, ce qui correspond à une conversion d'environ 28% d'azote en ammoniac.

## EXEMPLE 7

Le courant du gaz (33) est envoyé au séparateur (S9) via la ligne (34). Le séparateur (S9), un condenseur maintenu à -45°C, sert à fractionner le courant (34) en ammoniac (35) et en un mélange hydrogène et azote (41); ce dernier rejoint la ligne (31).

Le dioxyde de carbone, enlevé de l'effluent (21) par le séparateur (S5), est chauffé à -45°C puis transféré dans la conduite (20).

Le courant d'ammoniac (36) et le courant de dioxyde de carbone (37) sont chauffés à 0°C puis alimentent, séparément, un réacteur vertical en acier inoxydable. Le rapport NH$_3$/CO$_2$ est 4 et la température et la pression dans le réacteur sont de 190°C et 150 bars respectivement. Après un temps de séjour de 60 minutes, les produits ont été déchargés par le bas du réacteur. On a trouvé que 60% du carbamate d'ammonium, le produit initial de la réaction entre l'ammoniac et le CO$_2$, ont été convertis en urée.

TABLEAU N° 1

| EXEMPLE | 1 | 2 | 3 | 4 |
|---|---|---|---|---|
| Température du lit 1er réacteur °C | 880 | 880 | 880 | 880 |
| Température du 2ème réacteur | (a) | 850 | 850 | 850 |
| Débit (ml/min) $CH_4$ (e) | 1000 | 1000 | 1000 | 1000 |
| Débit (ml/min) $O_2$ (e) | 150 | 150 | 150 | 150 |
| Débit (ml/min) $C_2H_6$ (e) | 0 | 0 | 100 | 0 |
| Débit (ml/min) $C_2H_6$ introduit entre les 2 réacteurs | 0 | 0 | 0 | 100 |
| Débits (mol/h) (f) | | | | |
| $H_2$ | 0,093 | 0,160 | 0,243 | 0,318 |
| $O_2$ | 0,001 | 0,001 | 0,004 | 0,001 |
| CO | 0,038 | 0,041 | 0,069 | 0,043 |
| $CO_2$ | 0,105 | 0,101 | 0,109 | 0,103 |
| $C_2H_4$ | 0,091 | 0,133 | 0,219 | 0,292 |
| $C_2H_6$ | 0,091 | 0,042 | 0,066 | 0,104 |
| $C_3H_6$ | 0,005 | 0,009 | 0,013 | 0,010 |
| $C_3H_8$ | 0,003 | 0 | 0 | 0 |
| $CH_4$ | 1,969 | 1,981 | 2,213 | 2,032 |
| $H_2O$ | 0,500 | 0,505 | 0,455 | 0,499 |
| Total $(C_2 + C_3)$ | 0,190 | 0,184 | 0,298 | 0,406 |
| % conversion $CH_4$ (f) | 21,2 | 20,8 | (c) | 20,8(d) |
| % conversion $O_2$ (g) | 99,7 | 99,7 | | 99,7 |
| % conversion $C_2H_6$ ajouté(f) | (b) | (b) | (c) | 75,2(d) |
| Rapport oléfines/alcanes $C_2^+$ (f) | 1,0 | 3,4 | 3,5 | 2,9 |

* (a) pas utilisé ;

(b) pas applicable dans ces cas ;

(c) les conversions d'éthane et de méthane ne peuvent pas être directement calculées ;

(d) la conversion du méthane est égale à celle de l'exemple 2 ; l'augmentation de débit de méthane en sortie des réacteurs, dans l'exemple 4 par rapport à l'exemple 2, est imputée à la conversion partielle de l'éthane en méthane ;

(e) à l'entrée du premier réacteur ;

(f) à la ligne 40 ;

(g) à la ligne 39.

## Revendications

1. Procédé de production couplée d'oléfines et d'ammoniac à partir du gaz naturel, caractérisé en ce que :
   a) on sépare (S1) le gaz naturel en au moins deux fractions, une première fraction de gaz (4) enrichie en méthane et une deuxième fraction de gaz (3) enrichie en hydrocarbures supérieurs au méthane,
   b) on oxyde (U1) la première fraction de gaz provenant du gaz naturel (4) par un gaz renfermant de l'oxygène (12) en proportion de 0,05 à 0,4 mole par mole de méthane, en présence d'une masse de contact permettant le couplage oxydant du méthane en hydrocarbures supérieurs,
   c) on mélange l'effluent de l'étape (b) avec la fraction de gaz enrichie en hydrocarbures supérieurs (17) de l'étape (a) quand au moins 80% de l'oxygène moléculaire a déjà été consommé dans l'étape (b),
   d) on pyrolyse (U2) le mélange résultant de l'étape (c), de manière à déshydrogéner lesdits hydrocarbures supérieurs et à obtenir un mélange (18) renfermant des oléfines, de l'hydrogène et éventuellement de l'azote,
   e) on fractionne l'effluent de l'étape (d) afin d'obtenir une fraction riche en hydrogène (8),
   f) on ajuste les proportions d'hydrogène et d'azote de la fraction (8) riche en hydrogène, de manière à permettre la synthèse d'ammoniac, et
   g) on fait réagir (U5) le mélange (31) de l'étape (f), en présence d'un catalyseur pour la synthèse d'ammoniac, afin d'obtenir un courant de gaz renfermant de l'ammoniac.

2. Procédé selon la revendication 1, dans lequel l'azote de l'étape (f) provient au moins en partie du gaz naturel.

3. Procédé selon la revendication 1, dans lequel l'azote de l'étape (f) provient de la distillation de l'air, l'oxygène produit au cours de cette distillation étant alors utilisé comme source d'oxygène pour le procédé.

4. Procédé selon l'une des revendications 1 à 3, dans lequel le mélange de l'étape (c) est effectué lorsque au moins 95 % de l'oxygène moléculaire a déjà été consommé.

5. Procédé selon l'une des revendications 1 à 4, dans lequel, à l'étape (b), l'oxygène est en proportion de 0,05 à 0,2 mole par mole de méthane.

6. Procédé de production d'urée (U6) caractérisé en ce que l'on utilise de l'ammoniac (36), obtenu à l'étape (g) du procédé de l'une des revendications 1 à 5, en mélange avec du dioxyde de carbone (37) obtenu à l'une au moins des étapes (b) et (d).

## Patentansprüche

1. Verfahren zur gekoppelten Herstellung von Olefinen und Ammoniak aus Erdgas, dadurch gekennzeichnet, daß:

a) das Erdgas in mindestens zwei Fraktionen getrennt wird (S1), eine erste Gasfraktion (4), die mit Methan angereichert ist und eine zweite Gasfraktion (3), angereichert mit Kohlenwasserstoffen höher als Methan,

b) die erste Gasfraktion des Erdgases (4) mit einem Gas oxidiert wird (U1), das Sauerstoff (12) enthält, in einem Verhältnis von 0.05 bis 0.4 Mol pro Mol Methan, und zwar in Gegenwart einer Kontaktmasse, die die oxidative Kopplung von Methan zu höheren Kohlenwasserstoffen ermöglicht,

c) der Austrag des Schrittes (b) mit der Gasfraktion des Schrittes (a) gemischt wird, die mit höheren Kohlenwasserstoffen (17) angereichert ist, wenn mindestens 80 % an molekularem Sauerstoff bereits im Schritt (b) verbraucht wurden,

d) das Gemisch, das in Schritt (c) entsteht pyrolysiert (U2) wird, und zwar so, daß die erwähnten höheren Kohlenwasserstoffe dehydrogeniert werden und ein Gemisch (18) ergeben, das Olefine, Wasserstoff und eventuell Stickstoff enthält,

e) der Austrag des Schrittes (d) fraktioniert wird, damit schließlich eine wasserstoffreiche Fraktion (8) enthalten wird,

f) die Verhältnisse von Wasserstoff und Stickstoff der wasserstoffreichen Fraktion (8) so eingeregelt werden, daß die Synthese von Ammoniak möglich ist, und

g) das Gemisch (31) des Schrittes (f) in Gegenwart eines Katalysators zur Ammoniaksynthese zur Reaktion gebracht wird (U5), so daß schließlich ein Gasstrom erhalten wird, der Ammoniak enthält.

2. Verfahren gemäß Anspruch, in dem der Stickstoff für den Schritt (f) mindestens zum Teil aus dem Erdgas stammt.

3. Verfahren gemäß Anspruch, in dem der Stickstoff für den Schritt (f) durch Destillation von Luft gewonnen wird und der Sauerstoff, der im Laufe dieser Destillation gewonnen wird, dabei als Sauerstoffquelle für das Verfahren genutzt wird.

4. Verfahren gemäß einem der Ansprüche 1 bis 3, bei dem das Gemisch des Schrittes (c) eingesetzt wird, nachdem mindestens 95 % des molekularen Sauerstoffes bereits verbraucht worden sind.

5. Verfahren gemäß einem der Ansprüche 1 bis 4, bei dem im Schritt (b) Sauerstoff im Verhältnis von 0.05 bis 0.2 Mol pro Mol Methan eingesetzt wird.

6. Verfahren zur Herstellung von Harnstoff (U6), dadurch gekennzeichnet, daß der Ammoniak (36) eingesetzt wird, der im Schritt (g) eines Verfahrens gemäß einem der Ansprüche 1 bis 5 erhalten wird, gemischt mit Kohlenstoffdioxid (36), das bei mindestens einem der Schritte (b) und (d) erhalten wird.

## Claims

1. A process for the linked production of olefins and ammonia from natural gas, characterised by:

a) separating (S1) the natural gas into at least two fractions, a first gas fraction (4) enriched with methane and a second gas fraction (3) enriched with hydrocarbons higher than methane,

b) oxidising (U1) the first gas fraction coming from the natural gas (4) by means of a gas containing oxygen (12) in a proportion of from 0.05 to 0.4 mole per mole of methane, in the presence of a contact mass permitting oxidising coupling of the methane to provide higher hydrocarbons,

c) mixing the effluent from step b) with the gas fraction enriched with higher hydrocarbons (17) from step a) when at least 80% of the molecular oxygen has already been consumed in step b),

d) pyrolysing (U2) the mixture resulting from step c) so as to dehydrogenate said higher hydrocarbons and to obtain a mixture (18) containing olefins, hydrogen and possibly nitrogen,

e) fractionating the effluent from step d) in order to obtain a hydrogen-rich fraction (8),

f) adjusting the proportions of hydrogen and nitrogen of the hydrogen-rich fraction (8) so as to permit ammonia synthesis, and

g) reacting (U5) the mixture (31) of step f) in the presence of a catalyst for ammonia synthesis in order to obtain a flow of gas containing ammonia.

2. A process according to claim 1 wherein the nitrogen of step f) comes at least in part from the natural gas.

3. A process according to claim 1 wherein the nitrogen of step f) comes from the distillation of air, the oxygen produced in the course of the distillation operation then being used as a source of oxygen for the process.

4. A process according to one of claim 1 to 3 wherein the mixture of step c) is produced when at least 95% of the molecular oxygen has already been consumed.

5. A process according to one of claims 1 to 4 wherein, in step b) , the oxygen is in a proportion of from 0. 05 to 0. 2 mole per mole of methane.

6. A process for the production of urea (U6) characterised by using ammonia (36) obtained in step g) of the process of one of claims 1 to 5, mixed with carbon dioxide (37) obtained in one at least of steps b) and d).

Fig.1

Fig.2